(19) Europäisches Patentamt
European Patent Office
Office européen des brevets

(11) EP 1 696 233 A1

(12) # EUROPEAN PATENT APPLICATION
published in accordance with Art. 158(3) EPC

(43) Date of publication:
**30.08.2006 Bulletin 2006/35**

(21) Application number: **04807029.6**

(22) Date of filing: **08.12.2004**

(51) Int Cl.:
***G01N 33/50*** *(2006.01)* ***G01N 33/68*** *(2006.01)*
***G01N 21/77*** *(2006.01)*

(86) International application number:
**PCT/JP2004/018670**

(87) International publication number:
**WO 2005/057211 (23.06.2005 Gazette 2005/25)**

(84) Designated Contracting States:
**DE FR IT**

(30) Priority: **09.12.2003 JP 2003410415**

(71) Applicant: **SHISEIDO COMPANY, LTD.**
**Chuo-ku,**
**Tokyo 104-8010 (JP)**

(72) Inventors:
- **FUJITA Hiroshi,**
**c/o Shiseido Research Center**
**Yokohama-shi,**
**Kanagawa 2248558 (JP)**
- **KUBO, Sanae**
**Sagamihara-shi, Kanagawa 2290004 (JP)**
- **YASUDA, Masaaki,**
**c/o Shiseido Research Center**
**Yokohama-shi,**
**Kanagawa 2248558 (JP)**
- **HIRAO, Tetsuji,**
**c/o Shiseido Research Center**
**Yokohama-shi,**
**Kanagawa 2248558 (JP)**
- **KAWASOE, Tomoyuki,**
**c/o Shiseido Research Center**
**Yokohama-shi,**
**Kanagawa 2248558 (JP)**

(74) Representative: **Santarelli**
**14, avenue de la Grande Armée**
**Boîte Postale 237**
**75822 Paris Cedex 17 (FR)**

(54) **METHOD OF RATING HAIR DAMAGE WITH USE OF OXIDIZED PROTEIN OCCURRING IN HAIR AS INDICATOR**

(57) The present invention provides a method for evaluating hair damage. The method comprises specifically labeling the carbonyl group of an oxidized protein in hair with fluorescence and detecting the fluorescence. The damage can be caused by any of perm treatment, bleach treatment, treatment with an oxidation hairdye, combing, heat treatment, exposure to ultraviolet rays, and exposure to hypochlorous acid in swimming pools, or a combination thereof.

Fig.3

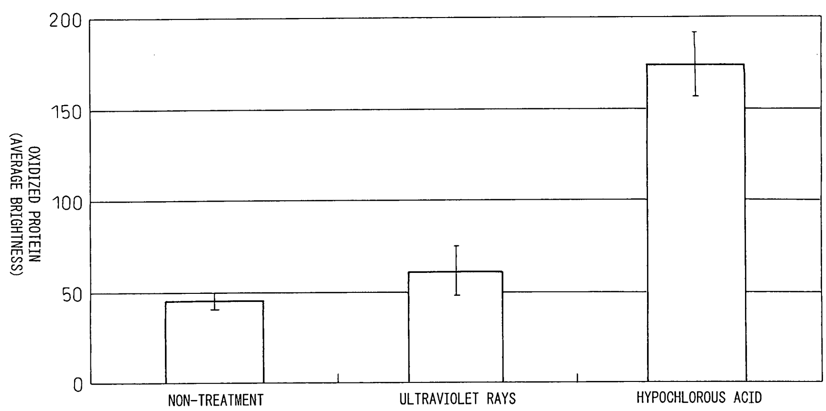


EP 1 696 233 A1

**Description**

Technical Field

The present invention provides a method, for evaluating a hair damage level, which comprises specifically labeling the carbonyl group of an oxidized protein in hair with fluorescence, and detecting the fluorescence, and a kit used for performing the method.

Background Art

Hair is damaged by various external factors such as perm treatment, bleach treatment, treatment with an oxidation hairdye, combing, heat treatment by a drier, exposure to ultraviolet rays, and exposure to hypochlorous acid in swimming pools. Hair cannot be repaired once damaged. In addition, the damage sometimes progresses over time. Accordingly, in order to keep one's hair healthy, it is naturally important to avoid any damage with daily care, and it is particularly important to recognize a damage level to prevent damage progression. As methods for evaluating a hair damage level, methods such as visual appreciation evaluation and physical property evaluation are known. For example, there are a method for analyzing the hair surface by scanning electron microscope (Swift J.A. et al., J. Society of Cosmetic Chemists (1972) Vol. 23, p. 695), a tensile test method for determining the mechanical strength of hair (Donald E.D. et al., J. Society of Cosmetic Chemists (1968) Vol. 19, p. 395), a method for determining a change in an inner structure of hair (Humphres W.T. et al., J. Society of Cosmetic Chemists (1972) Vol. 23, p. 359), and a method for determining the damage with a fluorescence microscope comprising labeling hair with a fluorescent substance (Tate M.L. et al., J. Society of Cosmetic Chemists (1993) Vol. 44, p. 347). They all have problems that a specific apparatus is required and operation is not simple.

Kokai (Jpn. Unexamined Patent Publication) No. 8-178920 and Kokai No. 8-271515 disclose a method for determining a hair damage level which comprises using an SH group, produced by cleavage of an S-S bond contained in large amounts in keratin produced, for example, in perm treatment, as an indicator for hair damage, and dyeing hair with fluorescence using a fluorescent substance which selectively labels the SH group, such as N-(9-acridinyl)maleimide and N-(7-dimethyl-amino-4-methylcoumarinyl)maleimide. In these methods, the damage can be detected with eyes without any use of a fluorescence microscope, and determination steps become simple. Generally, a perm treatment comprises cleaving an S-S bond in hair keratin by reduction treatment to produce an SH group, and oxidizing the SH group to an S-S bond. Bleach treatment is consisting of only oxidation treatment. However, both in the perm treatment and the bleach treatment, when the reaction excessively proceeds, the SH group and the S-S bond are changed to cysteic acid ($SO_3H$), which becomes a cause of hair damage. As cysteic acid does not react with a fluorescent substance which selectively labels the SH group, it is assumed that there are cases where the result, obtained by these methods using the SH group as an indicator for hair damage, does not accurately reflect the damage level.

Disclosure of the Invention

The object of the present invention is to provide an easy method for evaluating a hair damage level by which actual hair damage is more accurately reflected.

The present invention provides a method for evaluating hair damage. This method is characterized in that evaluation is conducted by specifically labeling the carbonyl group of an oxidized protein in hair with fluorescence and detecting the fluorescence. The damage can be caused by any of perm treatment of hair, bleach treatment, treatment with an oxidative hairdye, combing, heat treatment by a drier, exposure to ultraviolet rays, and exposure to hypochlorous acid in swimming pools, or a combination thereof.

In preferable examples, specific fluorescent labeling of the carbonyl group of an oxidized protein is carried out by reacting and binding a hydrazino-group-containing fluorescent substance to an oxidized protein. The hydrazino-group-containing fluorescent substance is preferably selected from the group consisting of fluorescein-5-thiosemicarbazide and dansylhydrazine.

In preferable examples, the above-described evaluation is performed under a fluorescence microscope. However, if the hydrazino-group-containing fluorescent substance is dansylhydrazine, fluorescence can be detected with the eye.

In another aspect of the invention, the present invention provides a kit used for a method for evaluating hair damage. The kit is characterized by containing a fluorescent substance for specifically labeling the carbonyl group of an oxidized protein with fluorescence.

A preferable fluorescent substance is a hydrazino-group-containing fluorescent substance. More preferably, the hydrazino-group-containing fluorescent substance is selected from the group consisting of fluorescein-5-thiosemicarbazide and dansylhydrazine.

The present invention provides an easy method for evaluating hair damage by which the actual hair damage is accurately reflected. In this evaluation method, damage caused by perm treatment, bleach treatment, treatment with an oxidative hairdye, combing, heat treatment by a drier, exposure to ultraviolet rays, and exposure to hypochlorous acid in swimming pools can be evaluated.

Brief Description of the Drawings

Fig. 1 shows a detection result of an oxidized protein in hair using fluorescein-5-thiosemicarbazide.
Fig. 2 shows a fluorescent micrograph showing an increased oxidized protein in hair by ultraviolet rays treatment and hypochlorous acid treatment.
Fig. 3 is a graph in which the result of Fig. 2 is diagramed in terms of average brightness.
Fig. 4 shows an oxidized protein in hair, represented by average brightness, increased by perm treatment and bleach treatment.
Fig. 5 shows an oxidized protein in hair, represented by average brightness, increased by perm treatment and bleach treatment, using dansylhydrazine as a fluorescent substance.
Fig. 6 shows a result of a quantitative experiment for hair damage by a medical ultraviolet fluorescent lamp.
Fig. 7a shows a photograph of hair damage by an artificial sunlight lamp, and Fig. 7b shows the result of the quantitative experiment.

Best Mode for Carrying Out the Invention

The present invention provides a method for evaluating a hair damage level which comprises specifically labeling the carbonyl group of an oxidized protein in hair with fluorescence and detecting the fluorescence, and a kit used for performing the method.

Hair (or a hair shaft) consists of hair cuticle coating the surface thereof, a cortex of a hair shaft which is inside thereof and accounts for main parts thereof, and hair medulla in the center part thereof. Particularly, hair cuticle imbricately coats the surface of hair from roots of hair to the tips thereof, and protects the inner parts. If hair cuticle is not damaged and is in a healthy condition, the hair looks glow. Hair mainly comprises 80 to 90 % of proteins, 1 to 8 % of lipids, 0.6 to 1.0 % of trace elements, and 12 to 13 % of water. There are a variety of proteins in hair, but the main protein is keratin. Into hair proteins including keratin, a carbonyl group is introduced due to oxidation caused by exposure to various factors such as a perm agent, a bleaching agent, a chemical oxidizing agent contained in an oxidative hairdye, ultraviolet rays, air pollutants, hypochlorous acid used in swimming pools, friction by combing, and heat by a drier. In the oxidation, there are a case where the $NH_2$ group of an amino acid residue such as Lys, Arg, and Pro of proteins is directly oxidized to produce a carbonyl group and a case where a lipid is oxidized to produce a lipid peroxide which will be further decomposed to produce a reactive aldehyde and bind to a protein.

Fluorescent substances specifically labeling the carbonyl group of an oxidized protein used in the present invention preferably include those having a hydrazino group $-NHNH_2$ which can bind to the carbonyl group of an oxidized protein. Examples of such fluorescent substances include fluorescein-5-thiosemicarbazide, dansylhydrazine, Texas Red hydrazide, and Lucifer Yellow hydrazide.

If these hydrazino-group-containing fluorescent substances are used, an oxidized protein can be detected, for example, by the following steps.

(1) hair is taken;
(2) the hair is reacted with a hydrazino-group-containing fluorescent substance in suitable buffer (for example, 100 mM MES-Na buffer (pH 5.5)) at room temperature for several hours (for example, an hour);
(3) after the reaction had been complete, the reactant is sufficiently washed with a suitable physiological solution (for example, phosphate-buffered saline (PBS)), and then an oxidized protein is detected by, for example, a fluorescence microscope; and
(4) micrographing is optionally carried out.

If a fluorescent substance, for example, dansylhydrazine, emitting visual fluorescence upon being irradiated with ultraviolet rays is used as a hydrazino-group-containing fluorescent substance, an oxidized protein labeled with fluorescence can be detected by eye without any use of a fluorescence microscope. Other fluorescent substances emitting visual fluorescence upon being irradiated with ultraviolet rays include N-(9-fluorenylmethoxycarbonyl)hydrazine (FMOC-hydrazine).

If a fluorescent substance, for example, dansylhydrazine, emitting visual fluorescence upon being irradiated with ultraviolet rays is used as a hydrazino-group-containing fluorescent substance, an oxidized protein can be detected, for example, by the following steps.

(1) hair is taken;
(2) the hair is reacted with a hydrazino-group-containing fluorescent substance in suitable buffer (for example, 100 mM MES-Na buffer (pH 5.5)) at room temperature for several hours (for example, an hour);
(3) after the reaction had been complete, the reactant is sufficiently washed with a suitable physiological solution (for example, phosphate-buffered saline (PBS)), and then it is irradiated with ultraviolet rays using a black light, etc.;
(4) an oxidized protein emitting fluorescence is observed by eye; and
(5) micrographing is optionally carried out.

As a specific fluorescent label for an oxidized protein, a combination of biotin hydrazide and fluorescently-labeled avidin can be used. Biotin hydrazide also has a hydrazino group, and can bind to the carbonyl group of proteins. In this case, biotin hydrazide is bonded to an oxidized protein, and then fluorescently-labeled avidin is bonded to the biotin hydrazide via a biotin-avidin bond whereby the oxidized protein is labeled with fluorescence. Biotin hydrazide is well known in industry of this field, and those manufactured and marketed by Pierce Corp. can be used. Fluorescein avidin can be used as a fluorescent avidin.

If a hydrazino-group-containing fluorescent substance is used, an oxidized protein can be detected, for example by the following steps.

(1) hair is taken;
(2) the hair is reacted with biotin hydrazide in suitable buffer (for example, 100 mM MES-Na buffer (pH 5.5)) at room temperature for several hours (for example, an hour);
(3) after the reaction had been complete, the reactant is sufficiently washed with a suitable physiological solution (for example, phosphate-buffered saline (PBS)), and then it is reacted with a fluorescently-labeled avidin at room temperature for several hours (for example, an hour);
(4) an oxidized protein is detected by, for example, fluorescence microscope; and
(5) micrographing is optionally carried out.

The kit of the present invention may contain reagents required for performing the above-described various types of evaluation methods, such as various buffers, other than the above-described fluorescent substances.

The inventors of the present invention found that, as described in the following examples, the amount of oxidized proteins in hair is correlated with a hair damage level to some degree. For example, oxidizing agents used for perm treatment are generally categorized into two groups of a hydrogen peroxide type and a sodium bromate type. It is well known that the hydrogen peroxide type has more oxidizing power and gives more damage to hair. When hair damage levels in hydrogen peroxide type perm treatment and those in sodium bromate type perm treatment were compared using an oxidized protein as an indicator, it was found that hair subjected to hydrogen peroxide type perm treatment was more damaged than that subjected to sodium bromate type perm treatment. Different from perm treatment, hair bleaching treatment only has one step of oxidizing hair. As reaction excessively proceeds, SH groups and S-S bonds are changed to cysteic acid ($SO_3H$), and damage cannot be accurately evaluated in the conventional method using an SH group as an indicator. However, when an oxidized protein was used as an indicator, and hair damage levels caused by bleach treatment and perm treatment were compared, it was found that hair subjected to bleach treatment was relatively more damaged than hair subjected to perm treatment. From these results, it was found that as hair damage level is increased, the amount of an oxidized protein in hair is increased. Accordingly, it is shown that in a method for evaluating hair damage using an oxidized protein as an indicator, actual hair damage can be accurately reflected.

Simple determination of hair damage using an oxidized protein as an indicator is realized by using the above-described detection method of an oxidized protein and a kit therefor. The method according to the present invention does not require steps of extracting proteins, electrophoresis, and Western-blotting. It can be performed only with a fluorescence microscope. If a dansylhydrazine is used as a fluorescent substance, an oxidized protein dyed with fluorescence can be evaluated by eye upon being irradiated with ultraviolet rays. Accordingly, in the above-identified detection method of an oxidized protein and with the kit therefor, useful information for hair evaluation can be obtained by simple operation and equipments. They can be performed easily at a place such as a cosmetic sales counter.

The present invention can be further specified with reference to the following examples.

Examples

Example 1

Detection of oxidized proteins in hair with fluorescein-5-thiosemicarbazide

One hair 5 cm from a hair root and one hair 5 cm from a hair tip (at about 20 cm from the root) were taken from nine subjects (who had not experienced any chemical hair treatment). They were dipped and washed in a washing agent (formulation 1) having the following composition at room temperature for 10 min and then, they were dipped and rinsed in water for one hour. After they were dried, they were fixed to slide glass with Aron Alpha. They were reacted with 100 mM MES-Na buffer (pH 5.5) containing 20 μM of fluorescein-5-thiosemicarbazide for one hour at room temperature. After the reaction had been complete, they were thoroughly washed in PBS, and were observed under a fluorescence microscope, and four images were captured for one sample. At digitalization, hair areas are separated from the background areas in the image, and an average brightness of hair was obtained.

Formulation 1 (Washing Agent)

| Name of the Ingredients | Blended Amounts (wt%) |
|---|---|
| Alscope NM (Toho Chemical Industry, Co., Ltd.) | 6.80 |
| coconut oil fatty acid amide | |
| propyl betaine solution (30 %) | 1.20 |
| citric acid | 0.06 |
| sodium benzoate | 0.09 |
| EDTA·3Na | 0.01 |
| ion-exchanged water | 91.84 |
| Total | 100.00 |

Fig. 1 shows the results. As is clear from Fig. 1, there was no individual difference in the samples taken from roots. On the other hand, there was remarkable individual difference in the samples taken from hair tips. It is assumed that the results reflect individual difference in hair care.

Example 2

Detection of oxidized proteins in hair with fluorescein-5-thiosemicarbazide

Three hairs 5 cm from hair tips were taken from one subject (who had not experienced any chemical hair treatment). They were dipped and washed in the washing agent (formulation 1) at room temperature for 10 min and then, they were dipped and rinsed in water for one hour. 200 J/cm$^2$ of ultraviolet rays (UVA) were irradiated to the first hair. The second hair was incubated in 0.2 mM sodium hypochlorite at 37 °C for 16 hours. No treatment was conducted for the third hair. The hairs, subjected to ultraviolet rays treatment and sodium hypochlorite treatment, were finally washed in the washing agent (formulation 1) at room temperature for 10 min and then, were dipped and rinsed in water for one hour. After they were dried, they were fixed to slide glass with Aron Alpha. They were reacted with 100 mM MES-Na buffer (pH 5.5) containing 20 μM of fluorescein-5-thiosemicarbazide at room temperature for one hour. After the reaction had been complete, they were thoroughly washed in PBS and were observed under a fluorescence microscope, and 12 images were captured for one sample. At digitalization, hair areas were separated from the background areas in the image, and an average brightness of hair was obtained.

Fig. 2 is a fluorescence micrograph of hair subjected to fluorescence treatment as described above. Fig. 3 shows an average brightness of each hair. As Figs. 2 and 3 show, increased oxidized proteins in hair were observed both in the ultraviolet ray treatment and the sodium hypochlorite treatment.

Example 3

Detection of oxidized proteins in hair with fluorescein-5-thiosemicarbazide

Four hairs 5 cm from hair tips were taken from one subject (who had not experienced any chemical hair treatment). They were dipped and were washed in the washing agent (formulation 1) at room temperature for 10 min and, then, they were dipped and rinsed in water for one hour. The first hair was subjected to bleach treatment. The second hair was subjected to (sodium bromate type) perm treatment. The third hair was subjected to (hydrogen peroxide type) perm treatment. No treatment was conducted for the fourth hair. In the bleach treatment, the agents "A", "B" and "C" of the following formulation 2 were mixed in a ratio of 4:6:1, and the hairs were dipped therein at room temperature for 30 min. These steps were repeated four times. In the (sodium bromate type) perm treatment, hairs were dipped in the solution of the following formulation 3-1 at 30 °C for 10 min, were washed in water for 30 sec, and were dipped in (sodium bromate type) solution 2 at 30 °C for 10 min. In the (hydrogen peroxide type) perm treatment, hairs were dipped in a solution of formulation 3-1 at 30 °C for 10 min, were washed in water for 30 sec, and were dipped in (hydrogen peroxide type) solution 3 at 30 °C for 5 min. Hairs, subjected to the bleach treatment and the perm treatment, were finally washed in the washing agent (formulation 1) at room temperature for 10 min and, then, were dipped and rinsed in water for one hour. After they were dried, they were fixed to slide glass with Aron Alpha. They were reacted with 100 mM MES-Na buffer (pH 5.5) containing 20 μM of fluorescein-5-thiosemicarbazide at room temperature for one hour. After the reaction had been complete, they were sufficiently washed in PBS, were observed under a fluorescence microscope, and eight images were captured for one sample. At digitalization, hair areas were separated from the background areas in the image, and an average brightness of hair was obtained.

Formulation 2 (Bleach Agent formulation for Bleach Damage)

Agent "A"

| Name of Ingredients | Blended Amount (wt%) |
|---|---|
| ammonia water (28 %) | 3.60 |
| ion-exchanged water | 96.40 |
| Total | 100.00 |

Agent "B"

| Name of Ingredients | Blended Amount (wt%) |
|---|---|
| hydrogen peroxide (30 %) | 20.00 |
| phosphoric acid (first grade) | 0.20 |
| disodium hydrogenphosphate (anhydrous) | 0.20 |
| tin sodium | 0.02 |
| methyl paraben | 0.05 |
| ion-exchanged water | 79.53 |
| Total | 100.00 |

Agent "C"

| Name of Ingredients | Blended Amount (wt%) |
|---|---|
| Persulfuric acid potassium salt | 74.22 |
| sodium metasilicate | 17.62 |
| ammonium sulfate | 1.91 |
| sodium pyrophosphate | 6.25 |
| Total | 100.00 |

Formulation 3 (Perm Agent Formulation for Perm Damage)

Agent 1

| Name of Ingredients | Blended Amount (wt%) |
|---|---|
| thioglycolic acid ammonium solution (50 %) | 12.90 |
| monoethanolamine | 0.90 |
| ammonium carbonate (first grade) | 2.80 |
| urea | 1.00 |
| EDTA·3Na | 0.10 |
| lauryldimethyl-aminoacetate betaine (40 %) | 0.30 |
| ion-exchanged water | 82.00 |
| Total | 100.00 |

Agent 2 (sodium bromate type)

| Name of Ingredients | Blended Amount (wt%) |
|---|---|
| sodium bromate solution (20 %) | 40.00 |
| potassium hydrogenphosphate | 0.30 |
| disodium hydrogenphosphate ($12H_2O$) | 0.20 |
| ion-exchanged water | 59.30 |
| Total | 100.00 |

Agent 3 (hydrogen peroxide type)

| Name of Ingredients | Blended Amount (wt%) |
|---|---|
| hydrogen peroxide solution (31 %) | 6.45 |
| phosphoric acid (1 %) | 2.00 |
| disodium hydrogenphosphate ($12H_2O$) | 0.03 |
| ion-exchanged water | 91.52 |
| Total | 100.00 |

Fig. 4 shows an average brightness of each hair. Both in the perm treatment and the bleach treatment, increased oxidized proteins in hair were observed. Interestingly, more oxidized proteins were observed in the hydrogen peroxide perm treatment than in the sodium bromate treatment. This is assumed to indicate that the oxidation level of hair is different depending on the type of perm treatment.

Example 4

Detection of oxidized proteins in hair with dansylhydrazine

Four hairs 5 cm from hair tips were taken from one subject (who had not experienced any chemical hair treatment). They were dipped and washed in the washing agent (formulation 1) at room temperature for 10 min and, then, they were dipped and rinsed in water for one hour. The first hair was subjected to bleach treatment. The second hair was subjected to (sodium bromate type) perm treatment. The third hair was subjected to (hydrogen peroxide type) perm treatment. No treatment was conducted for the fourth hair. In the bleach treatment, the agents "A", "B" and "C" of the following formulation 2 were mixed in a ratio of 4:6:1, and the hairs were dipped therein at room temperature for 30 min. These steps were repeated four times. In the (sodium bromate type) perm treatment, hairs were dipped in the solution of the following formulation 3-1 at 30 °C for 10 min, were washed in water for 30 sec, and were dipped in (sodium bromate type) solution 2 at 30 °C for 10 min. In the (hydrogen peroxide type) perm treatment, hairs were dipped in a solution 3-1 at 30 °C for 5 min, were washed in water for 30 sec, and were dipped in (hydrogen peroxide type) solution 3 at 30 °C for 10 min. Hairs, subjected to the bleach treatment and the perm treatment, were finally dipped and washed in the washing agent (formulation 1) at room temperature for 10 min, and were dipped and rinsed in water for one hour. After they were dried, they were fixed to slide glass with Aron Alpha. They were reacted with 100 mM MES-Na buffer (pH 5.5) containing 50 μM of dansylhydrazine at room temperature for one hour. After the reaction had been complete, they were thoroughly washed in PBS, and were observed under a fluorescence microscope, and four images were captured for one sample. At digitalization, hair areas were separated from the background areas in the image, and an average brightness of hair was obtained.

Fig. 5 shows an average brightness of each hair. Both in the perm treatment and the bleach treatment, increased oxidized proteins in hair were observed. Interestingly, more oxidized proteins were observed in the hydrogen peroxide type perm treatment than in the sodium bromate type treatment. This is assumed to indicate that the oxidation level of hair is different depending on the type of perm treatment.

Example 5

Using the present method, digitalization of hair damage in healthy black hair and bleached hair by ultraviolet rays was carried out.

(1) Experiment 1

As ultraviolet rays irradiation conditions, three medical ultraviolet fluorescence lamps (UV-B) [FL20S·E-30/DMR] were set at a height of 15 cm from the object (0.641 mW/cm$^2$).

Four standard samples were used:

(1) healthy black hair
(2) bleached hair 1 (healthy black hair subjected to bleach treatment for 15 min)
(3) bleached hair 2 (healthy black hair subjected to bleach treatment for 30 min)
(4) bleached hair 3 (healthy black hair subjected to bleach treatment for 30 min twice)

After the hairs of the above four standards were washed and were dried, they were divided into two groups of those not for irradiation and those for irradiation. Samples for irradiation were irradiated with ultraviolet rays for 70 hours. The irradiated samples were washed together with non-irradiated samples, and hair damage was evaluated using the present method. The obtained average brightness was used in the equation below to digitalize hair damage.

Equation:
Hair Sun Damage="Average Fluorescent Brightness of Irradiated Hair" - "Average Fluorescent Brightness of Non-irradiated Hair"

The result thereof is shown in Fig. 6.
Results:

Remarkable hair sun damage was observed in bleached hairs 2 and 3 having a reduced amount of melanin. As the amount of melanin in hair was reduced depending on a treatment period for bleaching, it is assumed that bleached hair has a reduced protection property against ultraviolet rays due to reduction of melanin.
It is found that the present test method is suitable for evaluating hair damage.
Experiments below were conducted using the most affected bleached hair 3.

(2) Experiment 2

Experiments were conducted for hairs subjected to bleaching for 30 min, twice.

As ultraviolet rays irradiation conditions, one artificial sunlight lamp SOLAX 500 W XC-500 B type (SELIC Ltd.) was set at a height of 40 cm from the object (UV strength: 54 W/m$^2$, UV irradiance level: 210 KJ/m$^2$: determined by a UV sensor [Toray Techno Co., Ltd.]).

Six irradiation standards are used (3, 6, 12, 24, 48 and 72 hours)

After the bleached hairs were washed and dried, they were divided into two groups of those not for irradiation and those for irradiation. Samples for irradiation were subjected to artificial sunlight lamp irradiation under the six standard conditions. The irradiated samples were washed together with non-irradiated samples, and hair damage was evaluated using the present method. The obtained average brightness was used in the equation below to digitalize hair sun damage.

Equation:
Hair Sun Damage="Average Fluorescent Brightness of Irradiated Hair" - "Average Fluorescent Brightness of Non-irradiated Hair"

The results thereof are shown in Fig. 7.
Results:

It was confirmed that hair sun damage was increased, depending on the irradiation period, by the artificial sunlight lamp. It was found that the present test method is suitable for evaluating hair damage by ultraviolet rays.
In consideration of effectiveness of an experiment, the effect of an antioxidizing agent was confirmed by irradiation with an artificial sunlight lamp for 12 hours.

(3) Experiment 3

Experiments were conducted for hair subjected to bleaching for 30 min, twice.
As ultraviolet rays irradiation conditions, one artificial sunlight lamp SOLAX 500 W XC-500 B type (SELILC LTD.) was set at a height of 40 cm from the object, and irradiation was conducted for 12 hours (UV strength: 54 $W/m^2$, UV irradiance level: 210 $KJ/m^2$: determined by a UV sensor [Toray Techno Co., Ltd.]).
Test Standards: Eight standards

(1) uncoated (ion-exchanged water)
(2) tea extract (0.1 %: Koeikogyo K. K.)
(3) lavender extract (0.1 %: Koeikogyo K. K.)
(4) grape seed extract (0.1 %: Kikkoman Corp.)
(5) tocopherol (0.001 %)
(6) tocopherol acetate (0.001 %)
(7) thiotaurine (0.001 %)
(8) glycerin (0.1 %)

After the bleached hairs were washed and dried, they were divided into two groups of those for non-irradiation and those for irradiation. Samples for irradiation were dipped in the above-described eight standard solutions for 15 min, and were taken out and dried. The dried samples were irradiated by an artificial sunlight lamp. The irradiated samples were washed together with non-irradiated samples, and hair damage was evaluated using the present method. The obtained average brightness was used in the equations 1 and 2 below to digitalize a protection effect against hair sun damage.

Equation 1:
Hair Sun Damage="Average Fluorescent Brightness of Irradiated Hair" - "Average Fluorescent Brightness of Non-irradiated Hair"

Equation 2:

Protection Effect against Hair Sun Damage (%) = ("Hair Sun Damage of Uncoated Hair" - "Hair Sun Damage of Coated Hair".) / "Hair Sun Damage of Uncoated Hair" x 100

For convenience, evaluation standards for a protection effect against hair sun damage were provided as below.

A: more than 80 %
B: 50 % or more and less than 80 %
C: 20 % or more and less than 50 %
D: less than 20 %

The effects of the drugs tested are described below. tea extract: B; lavender extract: B; grape seed extract: A; tocopherol: A; tocopherol acetate: A; thiotaurine: A; glycerin: D
Results:

It was found that the present method is also suitable for screening antioxidizing agents.

**Claims**

1. A method for evaluating hair damage comprising specifically labeling the carbonyl group of an oxidized protein in hair with fluorescence, and detecting the fluorescence.

2. The method according to claim 1, wherein the damage is caused by any of perm treatment, bleach treatment, treatment with an oxidative hairdye, combing, heat treatment, exposure to ultraviolet rays, and exposure to hypochlorous acid in swimming pools, or a combination thereof.

3. The method according to claim 1 or 2, wherein the specific fluorescence labeling of the carbonyl group of the oxidized protein is performed by acting and binding a hydrazino-group-containing fluorescent substance to the oxidized protein.

4. The method according to any one of claims 1 to 3, wherein the hydrazino-group-containing fluorescent substance is selected from the group consisting of fluorescein-5-thiosemicarbazide and dansylhydrazine.

5. The method according to any one of claims 1 to 4, wherein the evaluation is conducted under a fluorescence microscope.

6. The method according to claim 3, wherein the hydrazino-group-containing fluorescent substance is dansylhydrazine, and detection of the fluorescence is conducted by eye.

7. A kit used for a method for evaluating hair damage, comprising a fluorescent substance for specifically labeling the carbonyl group of an oxidized protein with fluorescence.

8. The kit according to claim 7, wherein the fluorescent substance is a hydrazino-group-containing fluorescent substance.

9. The kit according to claim 7 or 8, wherein the hydrazino-group-containing fluorescent substance is selected from the group consisting of fluorescein-5-thiosemicarbazide and dansylhydrazine.

Fig. 1

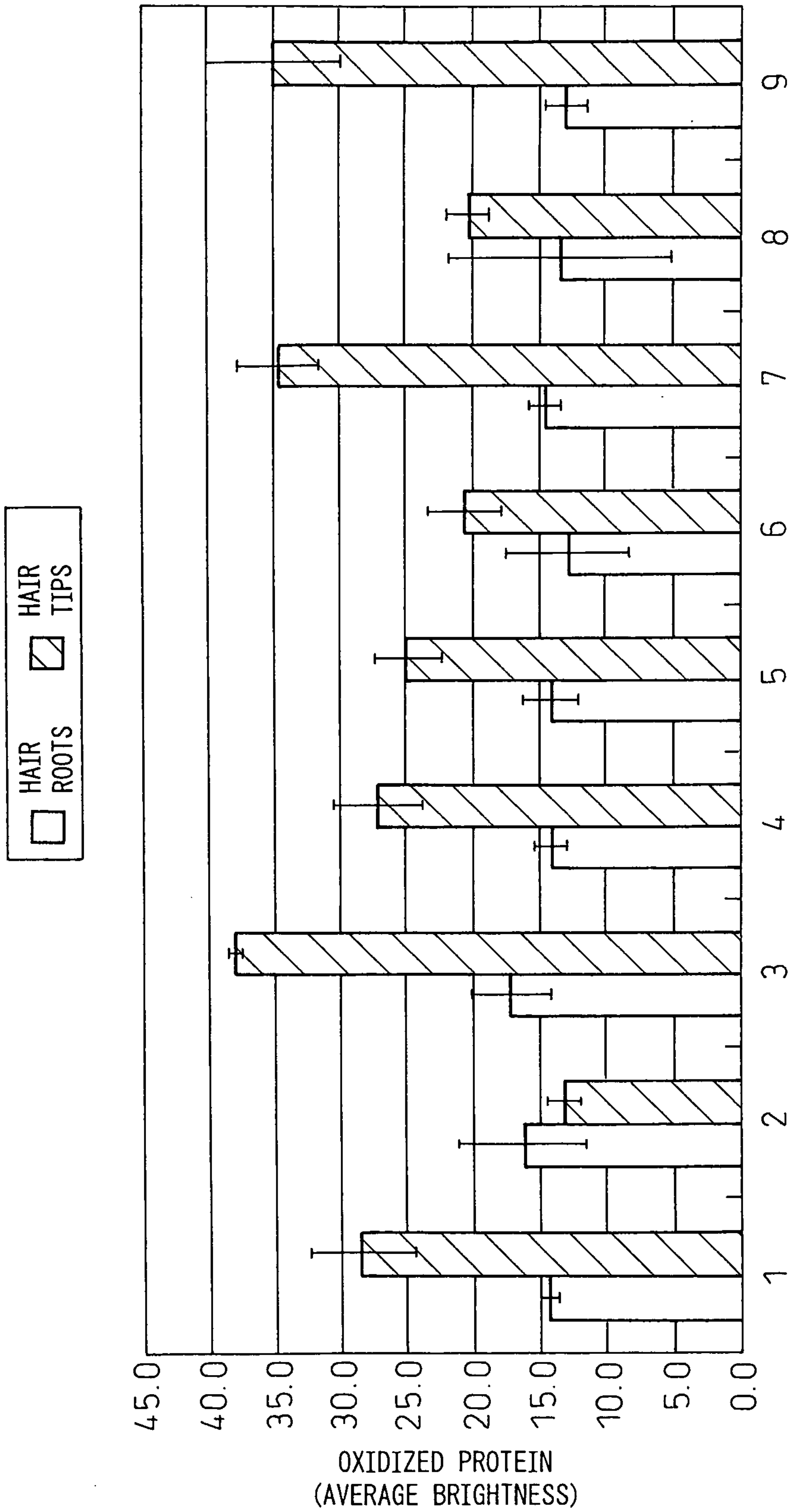
HAIR ROOTS
HAIR TIPS
45.0
40.0
35.0
30.0
25.0
20.0
15.0
10.0
5.0
0.0
1
2
3
4
5
6
7
8
9
OXIDIZED PROTEIN
(AVERAGE BRIGHTNESS)

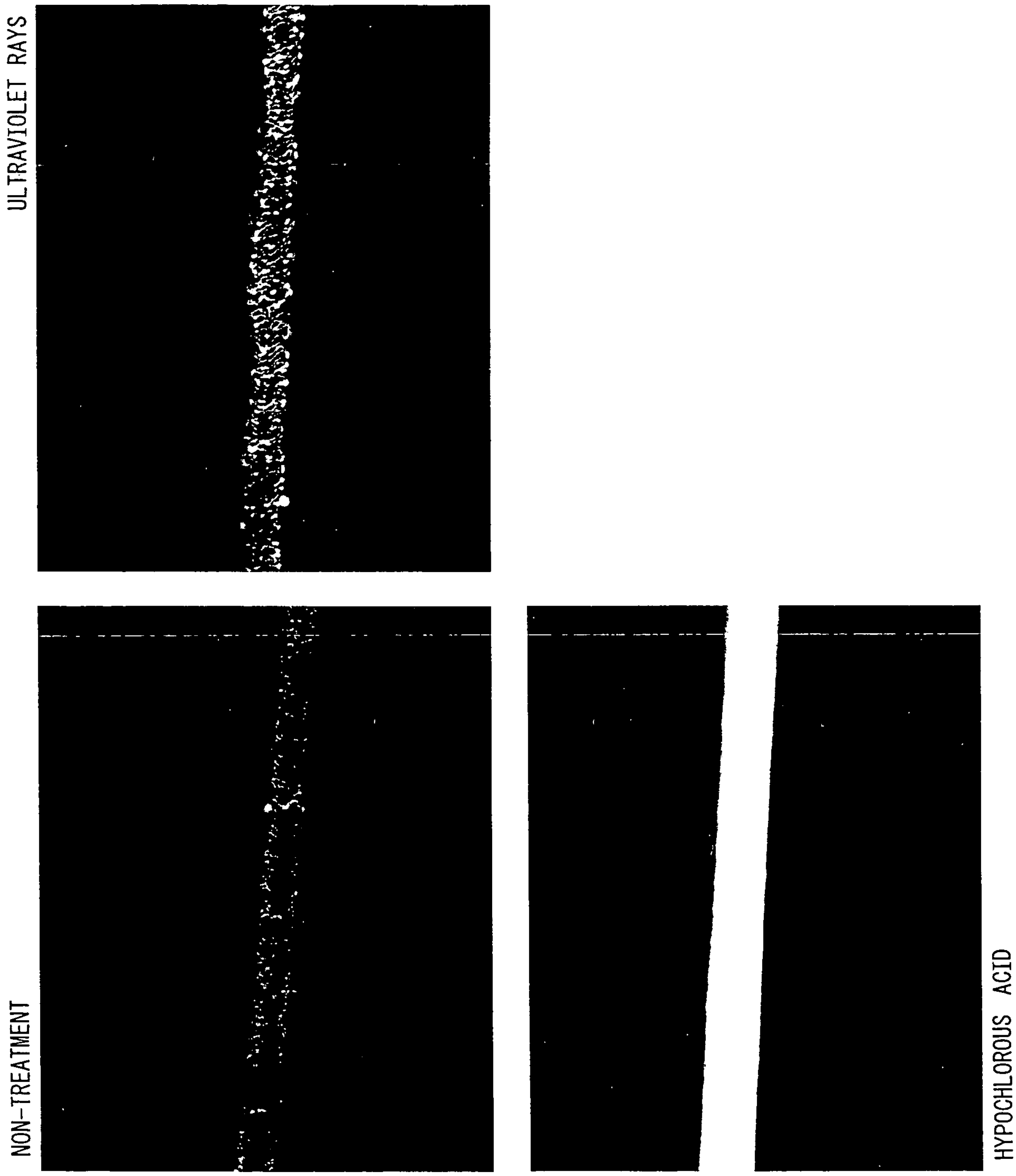


Fig.2

Fig.3

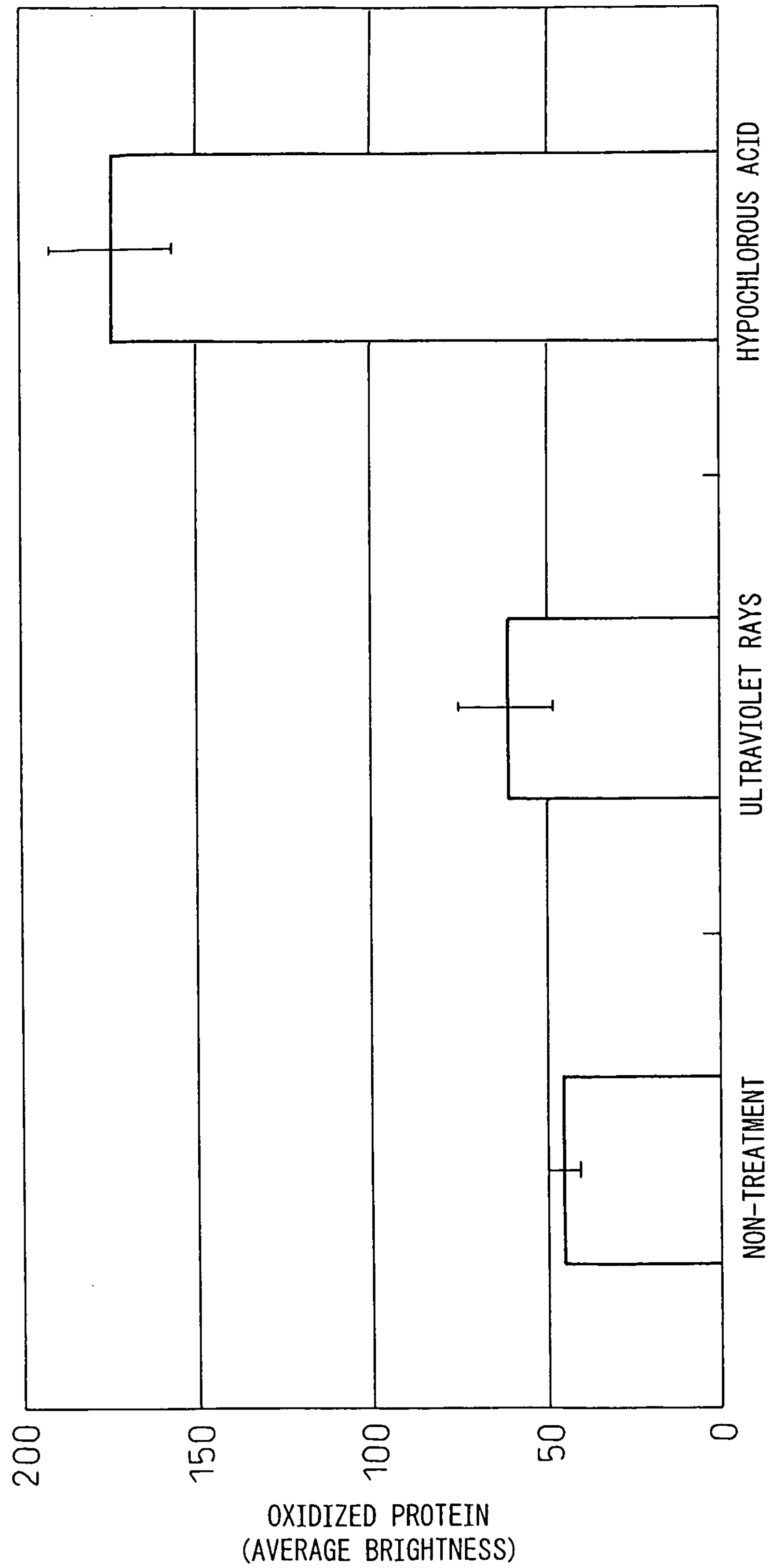
200
150
100
50
0
OXIDIZED PROTEIN
(AVERAGE BRIGHTNESS)
NON-TREATMENT
ULTRAVIOLET RAYS
HYPOCHLOROUS ACID

Fig.4

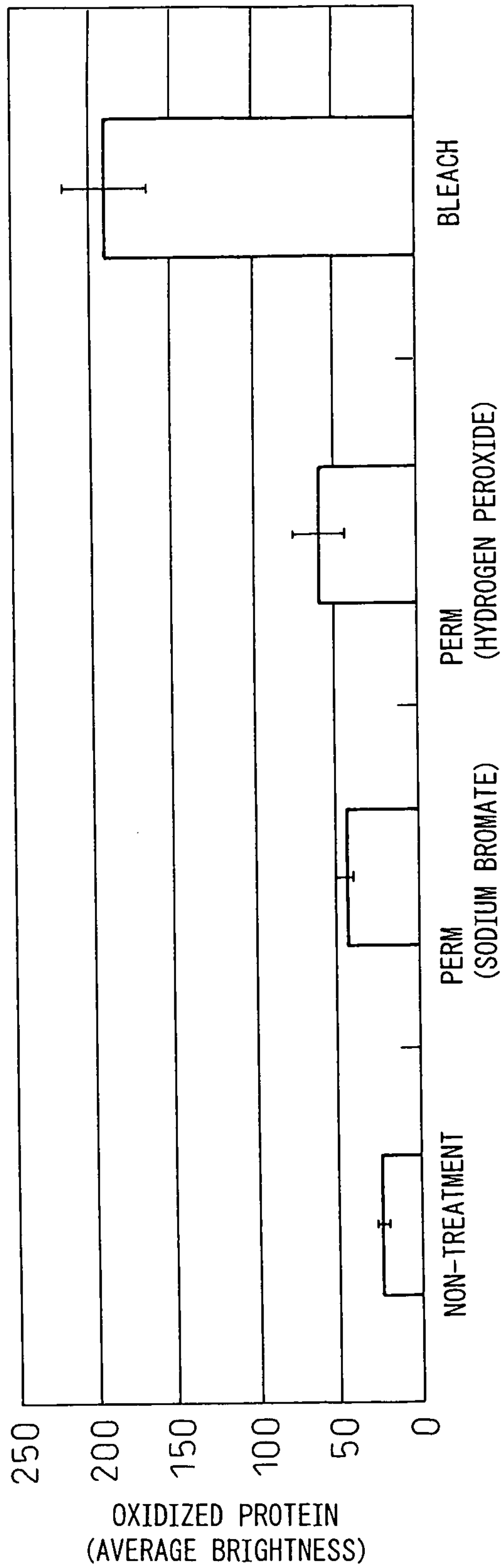
OXIDIZED PROTEIN
(AVERAGE BRIGHTNESS)
0
50
100
150
200
250
NON-TREATMENT
PERM
(SODIUM BROMATE)
PERM
(HYDROGEN PEROXIDE)
BLEACH

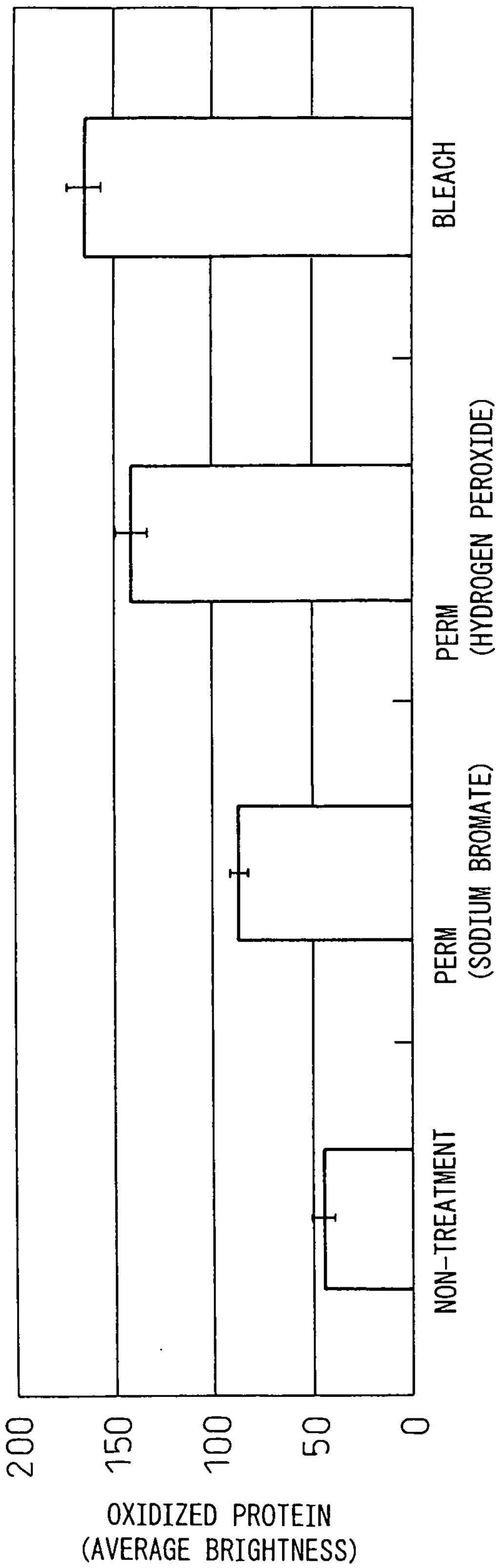
200
150
100
50
0
OXIDIZED PROTEIN
(AVERAGE BRIGHTNESS)
NON-TREATMENT
PERM
(SODIUM BROMATE)
PERM
(HYDROGEN PEROXIDE)
BLEACH

Fig.5

Fig.6

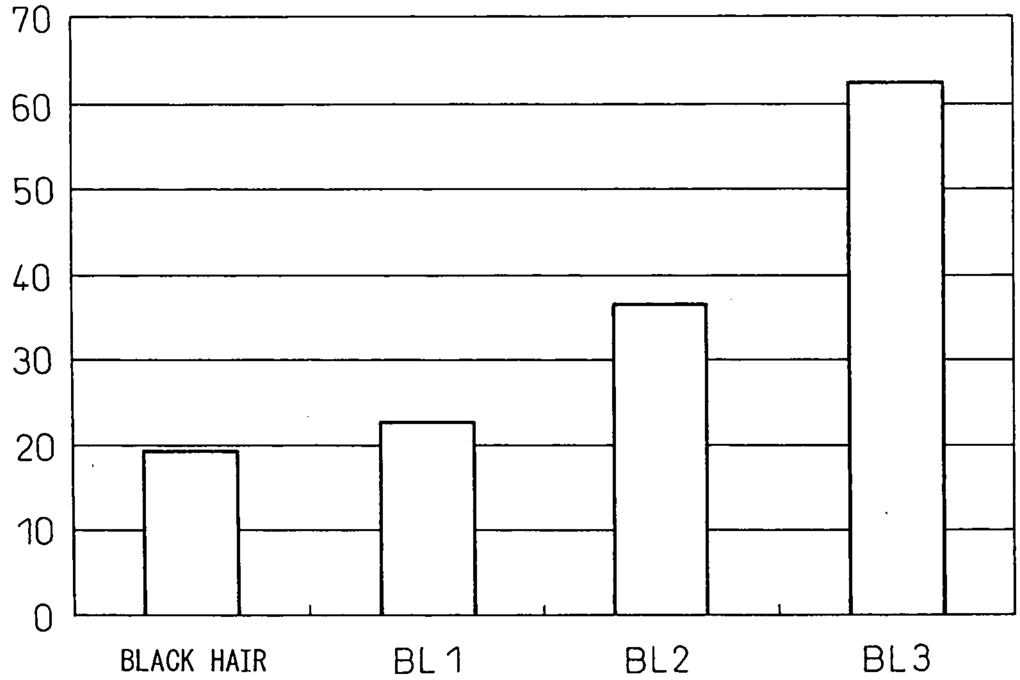

Fig.7a

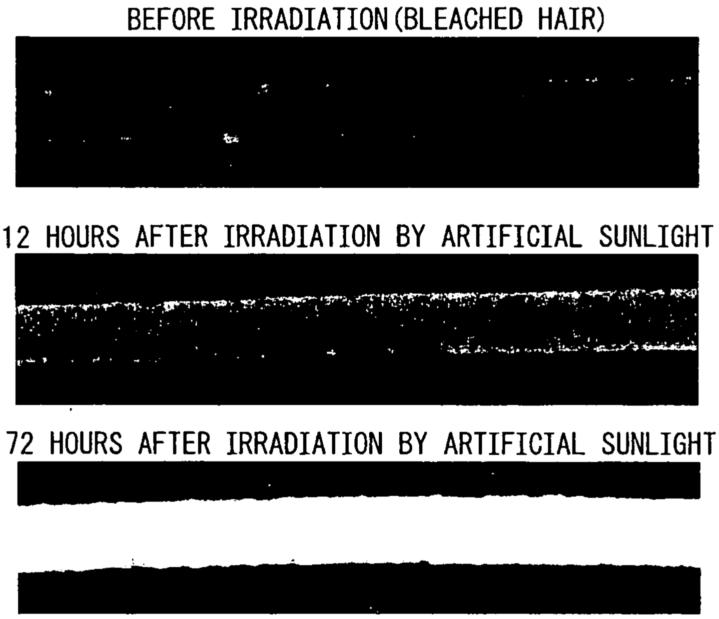
BEFORE IRRADIATION (BLEACHED HAIR)
12 HOURS AFTER IRRADIATION BY ARTIFICIAL SUNLIGHT
72 HOURS AFTER IRRADIATION BY ARTIFICIAL SUNLIGHT

Fig.7b

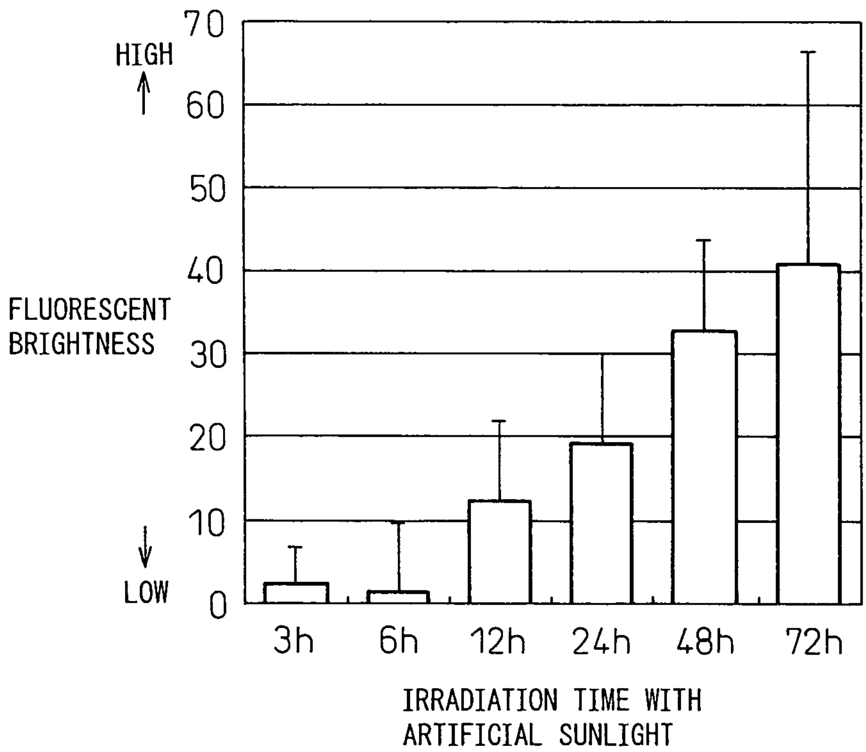
HIGH
LOW
FLUORESCENT BRIGHTNESS
70
60
50
40
30
20
10
0
3h
6h
12h
24h
48h
72h
IRRADIATION TIME WITH ARTIFICIAL SUNLIGHT

**INTERNATIONAL SEARCH REPORT**

International application No.
PCT/JP2004/018670

A. CLASSIFICATION OF SUBJECT MATTER
Int.Cl[7] G01N33/50, G01N33/68, G01N21/77

According to International Patent Classification (IPC) or to both national classification and IPC

B. FIELDS SEARCHED

Minimum documentation searched (classification system followed by classification symbols)
Int.Cl[7] G01N33/50, G01N33/68, G01N21/77

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)
JOIS

C. DOCUMENTS CONSIDERED TO BE RELEVANT

| Category* | Citation of document, with indication, where appropriate, of the relevant passages | Relevant to claim No. |
|---|---|---|
| A | JP 55-15099 A (L'Oreal), 01 February, 1980 (01.02.80), & IT 7968446 A & BE 877598 A & GB 2025613 A & DE 2928239 A & FR 2431126 A & US 4270919 A & CA 1109375 A & CH 640408 A | 1-9 |
| A | JP 8-271515 A (Matsushita Electric Works, Ltd.), 18 October, 1996 (18.10.96), (Family: none) | 1-9 |
| A | JP 9-127105 A (Kanebo, Ltd.), 16 May, 1997 (16.05.97), (Family: none) | 1-9 |

[X] Further documents are listed in the continuation of Box C.  [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 January, 2005 (20.01.05) | 08 February, 2005 (08.02.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No. PCT/JP2004/018670

C (Continuation). DOCUMENTS CONSIDERED TO BE RELEVANT

| Category* | Citation of document, with indication, where appropriate, of the relevant passages | Relevant to claim No. |
|---|---|---|
| A | JP 61-44353 A (Yuki Gosei Kogyo Co., Ltd.), 04 March, 1986 (04.03.86), (Family: none) | 1-9 |
| A | JP 7-224061 A (Director General of National Institute of Health Science), 22 August, 1995 (22.08.95), (Family: none) | 1-9 |

Form PCT/ISA/210 (continuation of second sheet) (January 2004)